# EUROPEAN PATENT APPLICATION

(11) **EP 4 523 662 A2**
(43) Date of publication of application: **19.03.2025**
(21) Application number: 24196005.3
(22) Date of filing: 22.08.2024
(51) Int. Cl.: A61F 2/42

(54) **REARFOOT REALIGNMENT AND STABILIZATION PLATE**

(30) Priority: 22.08.2023 US 202363578129 P; 24.01.2024 US 202463624429 P
(71) Applicant: Bruyn, James Mark, Austin, TX 78739 (US)
(72) Inventor: Bruyn, James Mark, Austin, TX 78739 (US)
(74) Representative: Müller Schupfner & Partner Patent- und Rechtsanwaltspartnerschaft mbB (Hamburg)

(57) **Abstract**

A surgical implant for use in subtalar joint motion limitation and having a semi-pliable body and a generally L- or J-shaped plate attached thereto. The plate may be in one or two pieces. A bar is formed on the end of the plate distal the semi-pliable body. A plurality of bores extend through the plate and bar to allow for the attachment of the implant to a subtalar joint bone.

## Description

### FIELD OF THE INVENTION

The present invention relates to a surgical implant and, more particularly, to a laterally stabilized subtalar joint motion altering implant with a dual purpose calcaneal plate.

### BACKGROUND OF THE INVENTION

Flexible painful flatfoot deformity is a condition that has been treated surgically for greater than 50 years. Previous subtalar joint (STJ) stabilizing and calcaneal lengthening procedures tended to result in dislocation and subluxation, which the plate of the present invention is intended to prevent.

### SUMMARY OF THE INVENTION

In one aspect, the present invention relates to an implant for use in STJ motion limiting procedures.

In another aspect, the present invention relates to a specialized osteotome for use in STJ motion limiting procedures.

### BRIEF DESCRIPTION OF THE DRAWINGS

Fig. 1 is a side view of one embodiment of the implant of the present invention.
Fig. 2 is a top view of one embodiment of the implant of the present invention.
Fig. 3 depicts a side view of the subtalar joint facets with sections to be removed indicated.
Fig. 4 depicts a side view of the subtalar joint with an embodiment of the implant of the present invention installed.
Fig. 5 depicts a top view of the subtalar joint with the implant of the present invention installed.
Fig. 6 is a perspective view of the embodiment of Fig. 4.
Fig. 7 depicts another embodiment of the implant of the present invention.
Fig. 8 is another view of the embodiment of Fig. 7.
Fig. 9 depicts yet another embodiment of the implant of the present invention.
Fig. 10 is another view of the embodiment of Fig. 9.
Fig. 11 depicts an osteotome for use during installation of the implant of the present invention.
Fig. 12 is another view of the osteotome of Fig. 11.

### DETAILED DESCRIPTION OF PREFERRED EMBODIMENTS

Embodiments of the invention are described more fully hereafter with reference to the accompanying drawings. Elements that are identified using the same or similar reference characters refer to the same or similar elements which perform the same functions across various embodiments. The various embodiments of the invention may, however, be embodied in many different forms and should not be construed as limited to the embodiments set forth herein. Rather, these embodiments are provided so that this disclosure will be thorough and complete, and will fully convey the scope of the invention to those skilled in the art.

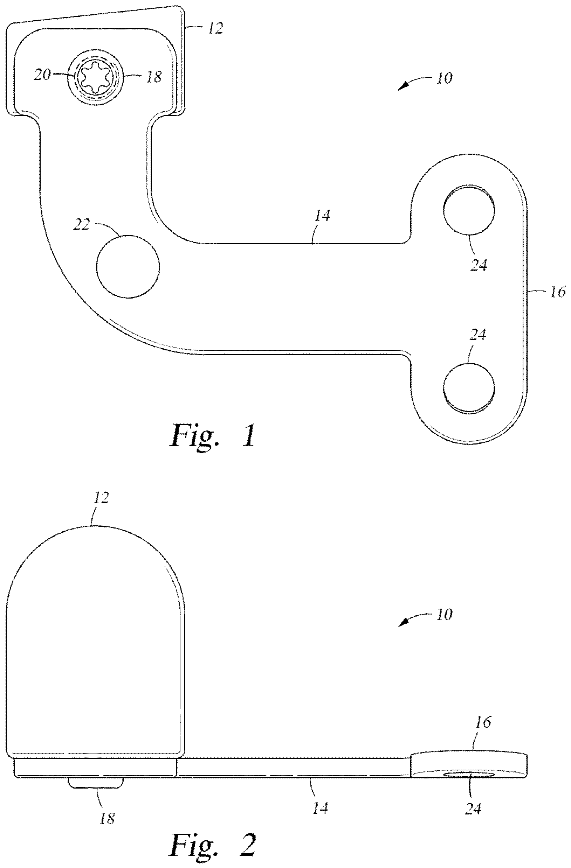

Turning first to Figs. 1 and 2, there is shown generally as 10 one embodiment of the present invention. The implant 10 has a generally trapezoidal body 12. It will be appreciated though that body 12 may be rectangular or vary somewhat in shape. Some of the embodiments depicted herein show a rectangular body 12. A plate 14 is affixed to a peripheral surface of body 12. In a preferred embodiment, plate 14 is generally L- or J-shaped. The end of plate 14 is formed into a bar 16. In a preferred embodiment, body 12 is formed from a semi-pliable material, more preferably, polypropylene or polyetheretherketone (PEEK). Plate 14 and bar 16 are preferably formed from a rigid material, more preferably they are formed from titanium or stainless steel. In a preferred embodiment, plate 14 and bar 16 are of unitary construction. As shown in Fig. 1, plate 14 is attached to body 12 via pin 18 which extends through bore 20 in plate 14. The exact attachment mechanism between body 12 and plate 14 may vary though. Plate 14 includes one or more bores 22 for receiving screws 26. Bar 16 includes one or more bores 24 for receiving screws 28.

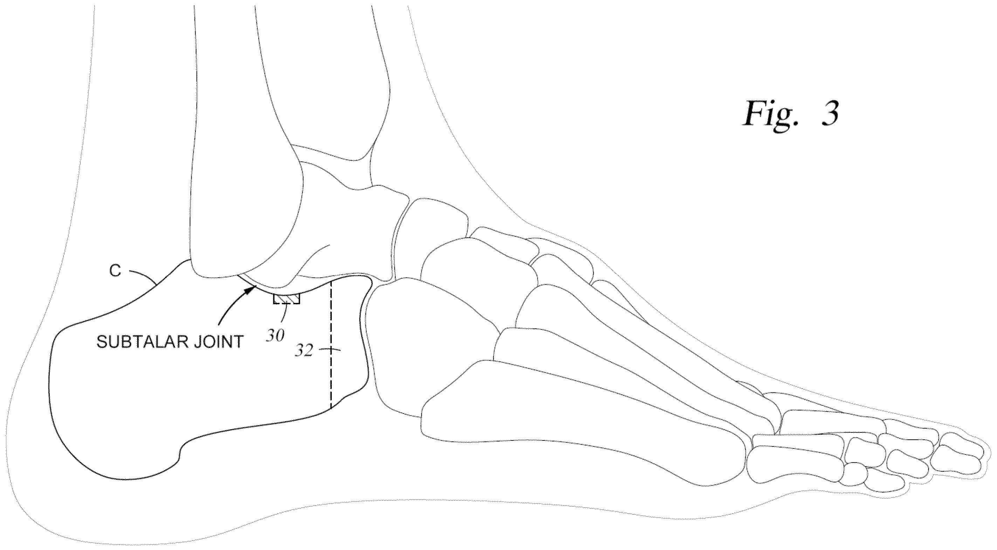

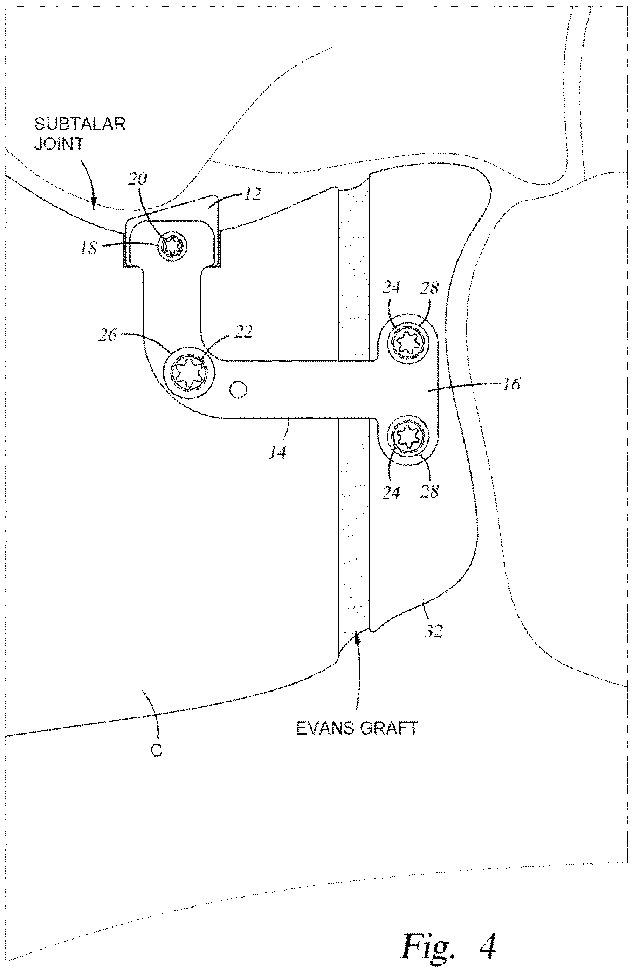

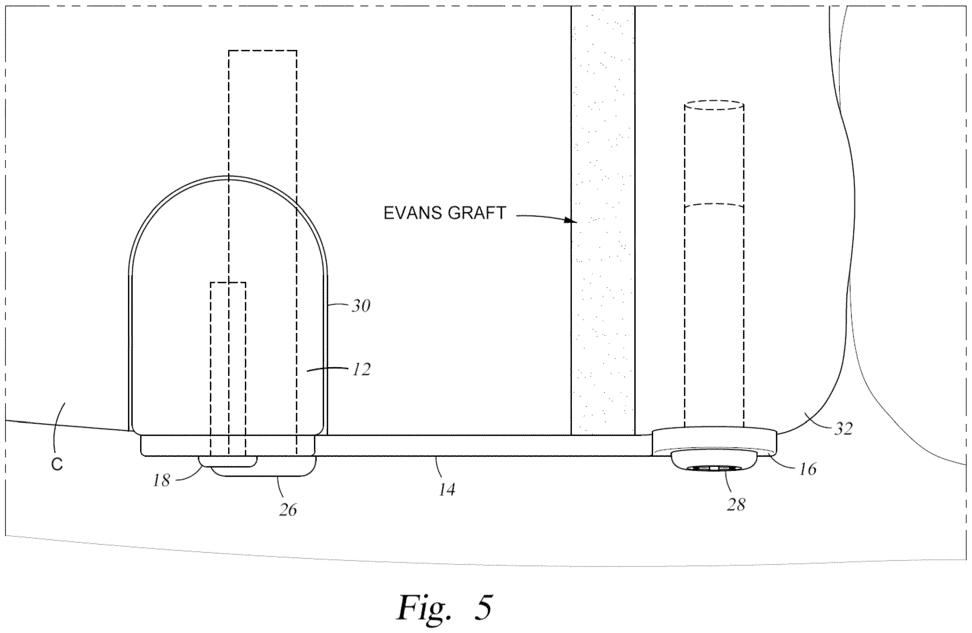

Figs. 3-5 depict the use of the implant of the present invention. Fig. 3 shows the subtalar joint STJ. The bone of the subtalar joint STJ is cut in two places (shown in dotted lines). A portion of the surface 30 is removed and about 2 cm of the anterior portion 32 of the calcaneus C is removed. The implant 10 is positioned with the body 12 resting where the portion 30 was removed. Plate 14 extends along the side of the calcaneus C and is affixed thereto by one or more screws 26. Bar 16 is affixed to the anterior portion 32 by one or more screws. The screws used in plate 14 and bar 16 can be from 2.7 mm to 3.5 mm. Anterior portion 32 is also reattached to the calcaneus C by an Evans graft or other means well known to those skilled in the art.

It will be appreciated by those skilled in the art that the drawings are not to scale and that the sizes of the components of implant 10 can vary to accommodate different sized feet. For example, without being limiting, body 12 may be a bit larger or the length of bar 16 may be shorter. Likewise, the screws used may vary in length depending on the needs of the patient.

The implant of the present invention provides multiple advantages over the prior art. The semi-pliable body 12 provides the support and flexibility needed in the subtalar joint. Rigid plate 14 and bar 16 provide added stabilization and support to the implant and foot. The implant also allows for the use of smaller screws. Prior art surgical techniques often use 6.5 mm or larger screws whereas the present invention can work with smaller screws, as low as 2.7 - 3.5 mm. The screws and screw holes provide polyaxial locking of 0° to 15° per side.

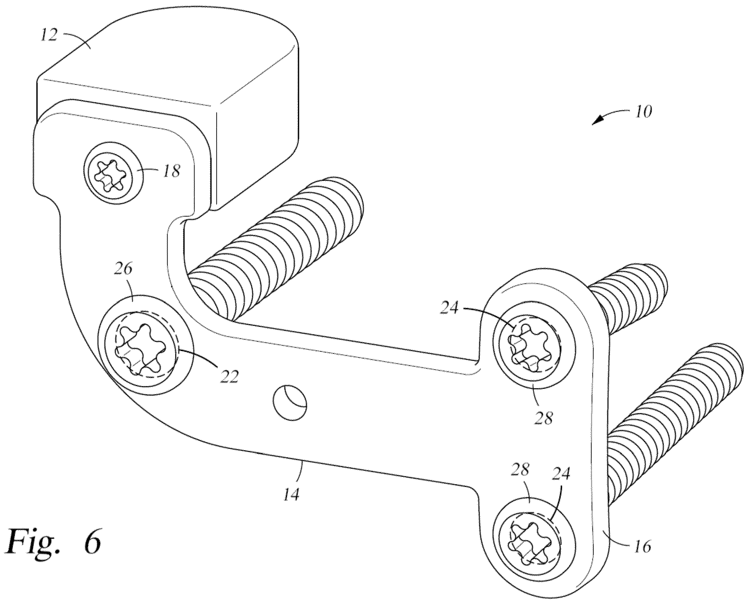

Fig. 6 shows a perspective view of implant 10 with the screws. As depicted in Fig. 6, body 12 is rectangular rather than trapezoidal.

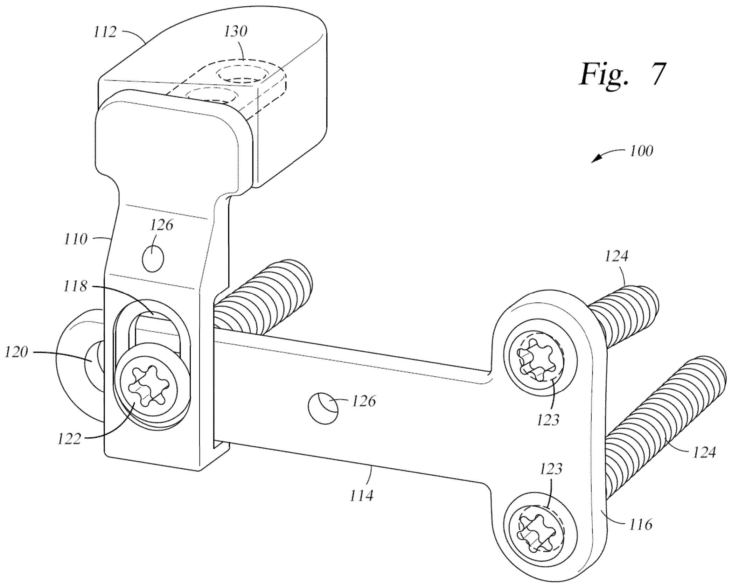

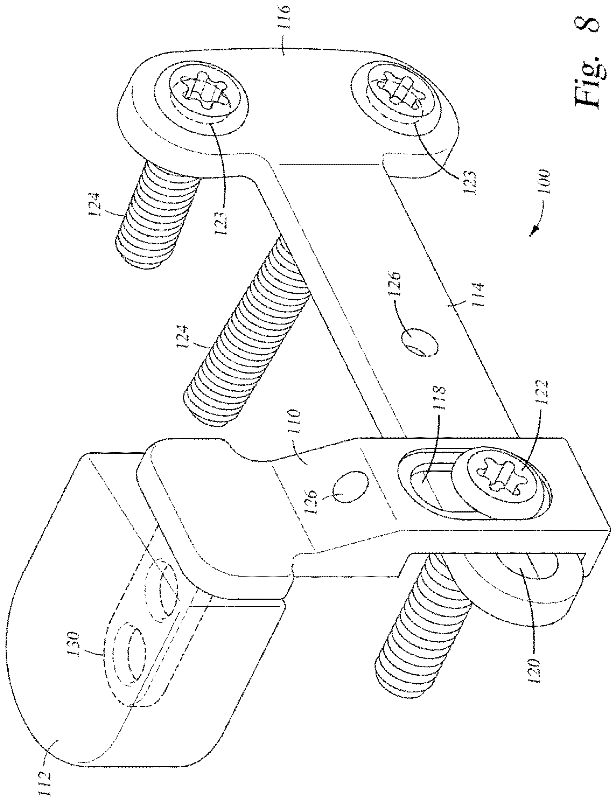

Turning to Figs. 7 and 8, there is shown another embodiment of the implant of the present invention. The implant 100 of Figs. 7 and 8 is similar to implant 10, but the plate is in two pieces. Implant 100 has a generally trapezoidal body 112. It will be appreciated though that body 112 may be rectangular or vary somewhat in shape. A plate 110 is affixed to the peripheral surface of body 112. A cross-plate 114 is affixed to plate 110. In a preferred embodiment, plate 110 and cross-plate 114 form a generally L-shaped configuration.

The end of plate 114 is formed into a bar 116. In a preferred embodiment, body 112 is formed from a semi-pliable material, more preferably, polypropylene or polyetheretherketone (PEEK). Plate 110, cross-plate 114, and bar 116 are preferably formed from a rigid material, more preferably they are formed from titanium or stainless steel. In a preferred embodiment, cross-plate 114 and bar 116 are of unitary construction. Plate 110 is attached to body 112 via leg 130 which extends through body 112. It will be appreciated though that plate 110 may be attached to body 112 via one or more pins, as in the embodiment of Figs. 1-6. Likewise, in the embodiments of Figs. 1-6 body 12 may be attached to plate 14 via a leg, as in the embodiment of Fig. 7. Plate 110 includes one or more bores 118. In a preferred embodiment, plate 110 includes one elongated bore 118. Cross-plate 114 includes one or more bores 120. In a preferred embodiment, cross-plate 114 includes one elongated bore 120. Bores 118 and 120 are positioned to be in register with one another such that a screw 122 can extend through both. In a preferred embodiment, the elongated shape of bores 118 and 120 allows for adjustability between plate 110 and cross-plate 114. Bar 116 includes one or more bores 123 for receiving screws 124. One or more pin holes 126 may extend through plate 110 or cross-plate 114 for receiving pins to further secure implant 100.

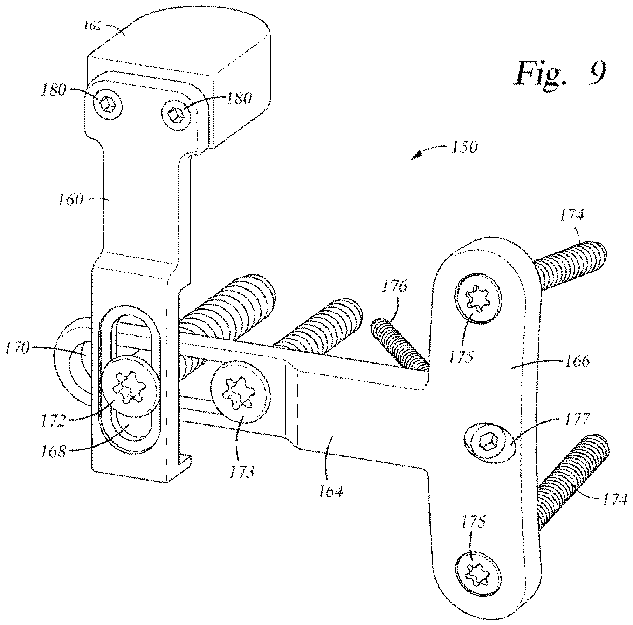

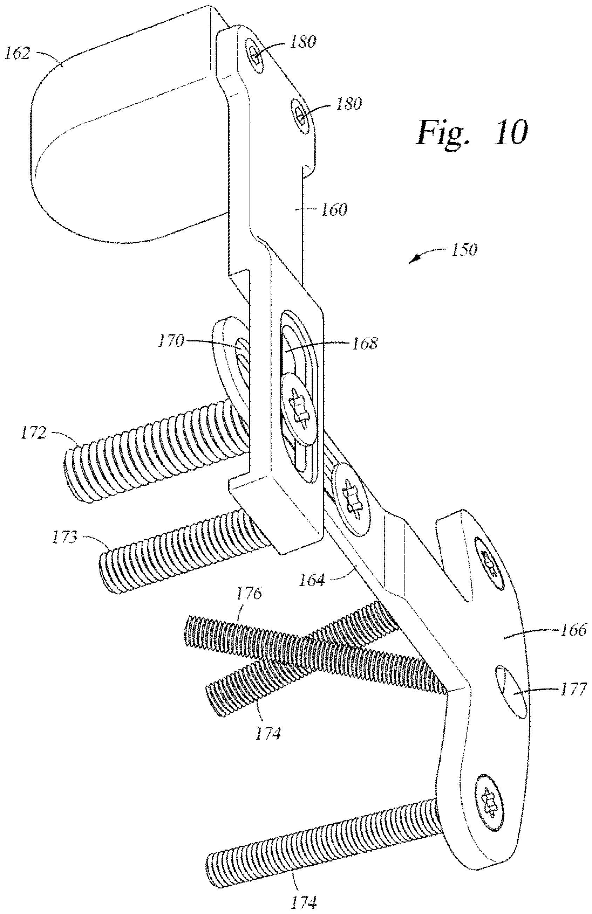

Turning to Figs. 9 and 10, there is shown yet another embodiment of the implant of the present invention. Implant 150 is similar to implant 100 in that it is a two-piece construction. Implant 150 has a generally rectangular body 162. It will be appreciated though that body 162 may be trapezoidal or vary somewhat in shape. A plate 160 is affixed to the peripheral surface of body 162. A cross-plate 164 is affixed to plate 160. In a preferred embodiment, plate 160 and cross-plate 164 form a generally L-shaped configuration. The end of plate 164 is formed into a bar 166. In a preferred embodiment, body 162 is formed from a semi-pliable material, more preferably, polypropylene or polyetheretherketone (PEEK). Plate 160, cross-plate 164, and bar 166 are preferably formed from a rigid material, more preferably they are formed from titanium or stainless steel. In a preferred embodiment, cross-plate 164 and bar 166 are of unitary construction. Plate 160 is attached to body 162 via pins 180 which extend through body 162. It will be appreciated though that plate 160 may be attached to body 162 via a metal leg or the like, as in the embodiment of Figs. 7-8. Plate 160 includes one or more bores 168. In a preferred embodiment, plate 160 includes one elongated bore 168.

Cross-plate 164 includes one or more bores 170. In a preferred embodiment, cross-plate 164 includes one elongated bore 170. Bores 168 and 170 are positioned to be in register with one another such that a screw 172 can extend through both. Implant 150 has longer bores than in implant 100, and may also, if needed, have a longer plate and / or cross-plate. Thus, as shown in Figs. 9 and 10, a second screw 173 extends through bore 170, though not bore 168. The elongated shape of bores 168 and 170 allows for adjustability between plate 160 and cross-plate 164. The greater size of the bores allows for additional screws, e.g., screw 173 to provide a more secure fastening of implant 150. It will be appreciated that while an extra screw 173 is shown with respect to bore 170, such may also be used in bore 168 if needed. Bar 166 includes one or more bores 175 for receiving screws 174. If desired, bar 166 may include a third bore 177 for receiving screw 176. Bore 177 may be angled such that screw 177 is positioned at an angle relative to screws 172, 173.

The process of installing implants 100 and 150 is the same as for implant 10, though the exact number and length of screws may vary depending upon the patient.

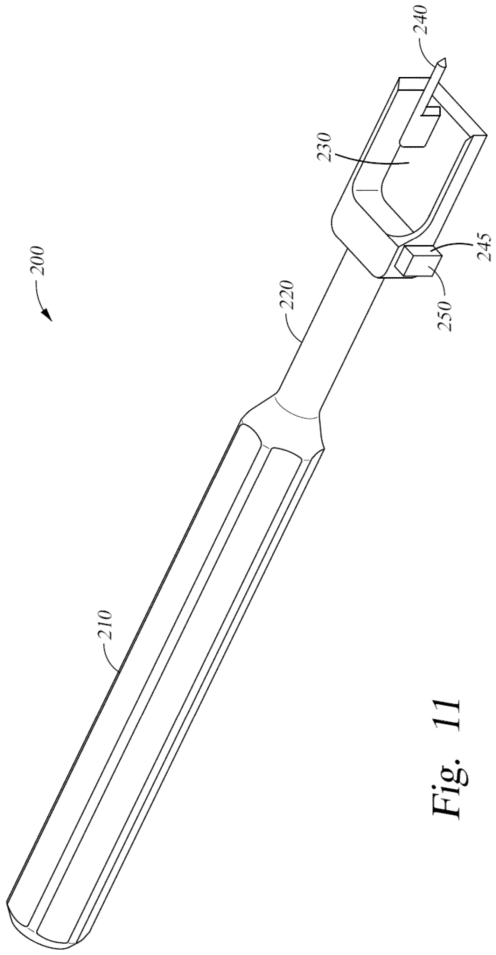

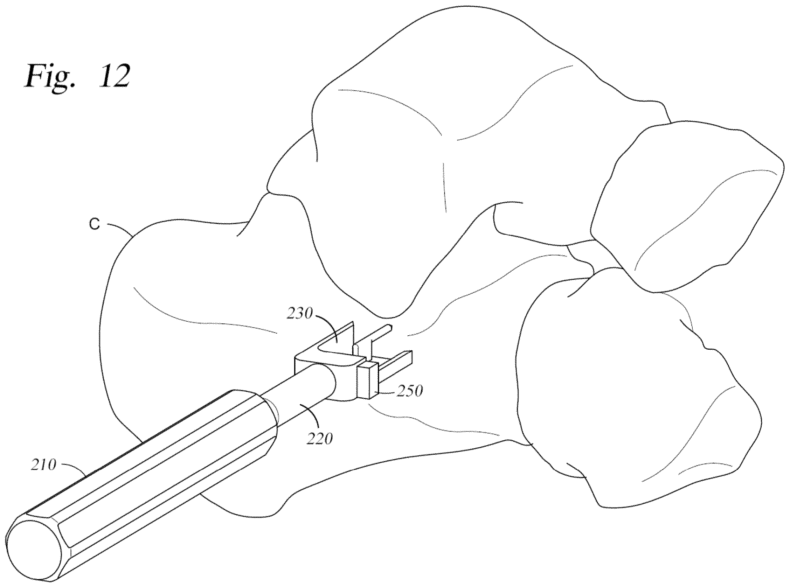

Turning now to Figs. 11 and 12, there is shown an osteotome for use during installation of implants, 10, 100, or 150. Osteotome 200 has an elongated handle 210. Extending from handle 210 is a shaft 220 and a blade 230. Positioned on blade 230 is a pin 240. Extending alongside blade 230 is a projection 245. The lateral surface of projection 245 is a guide surface 250 which provides a cutting guide for the Evans osteotomy. In use, osteotome 200 is held in the desired position against the calcaneus C. Pin 240 punctures calcaneus C to hold osteotome 200 in place. The surgeon can then remove the anterior portion 32 of calcaneus C using guide surface 250 to ensure a straight cut at the correct position.

The present invention provides multiple advantages over the prior art. This invention allows the surgeon a means of stabilizing and fixating two separate procedures simultaneously. This results in a significant reduction in surgical time, risks, and cost to the patient. This is accomplished by using a plate that provides both intra as well as extra articular fixation and stabilization when performing an "Evans" calcaneal osteotomy.

Although specific embodiments of the invention have been described herein in some detail, this has been done solely for the purposes of explaining the various aspects of the invention and is not intended to limit the scope of the invention as defined in the claims which follow. Those skilled in the art will understand that the embodiment shown and described is exemplary, and various other substitutions, alterations and modifications, including but not limited to those design alternatives specifically discussed herein, may be made in the practice of the invention without departing from its scope.

## Claims

1. A surgical implant for use in limiting exogenous subtalar joint motion, comprising:
a body having a peripheral surface;
a plate having a first end and a second end, said first end being affixed to the peripheral surface of said body, said plate being generally L- or J-shaped;
a bar formed on said second end of said plate;
one or more bores formed through said plate; and
one or more bores formed through said bar.

2. The surgical implant of claim 1, wherein said body is formed from a semi-pliable material.

3. The surgical implant of claim 1 or 2, wherein said body is formed from polypropylene or polyetheretherketone.

4. The surgical implant of one or more of claims 1 to 3, wherein said plate and bar are formed of a rigid material.

5. The surgical implant of one or more of claims 1 to 4, wherein said plate and said bar are formed from titanium or stainless steel.

6. The surgical implant of one or more of claims 1 to 5, wherein said plate and said bar are of unitary construction.

7. A surgical implant for use in limiting exogenous subtalar joint motion, comprising:
a body having a peripheral surface;
a first plate having a first end and a second end, said first end of said first plate being affixed to said body;
a second plate having a first end and a second end, said first end of said second plate being attachable to the second end of said first plate;
a bar formed on said second end of said second plate;
one or more bores formed through said first plate;
one or more bores formed through said second plate; and
one or more bores formed through said bar.

8. The surgical implant of claim 7, wherein said body is formed from a semi-pliable material.

9. The surgical implant of claim 7 or 8, wherein said body is formed from polypropylene or polyetheretherketone.

10. The surgical implant of one or more of claims 7 to 9, wherein said first plate, second plate, and bar are formed of a rigid material.

11. The surgical implant of one or more of claims 7 to 10, wherein said first plate, second plate, and bar are formed from titanium or stainless steel.

12. The surgical implant of one or more of claims 7 to 11, wherein said second plate and said bar are of unitary construction.

13. An osteotome for use in installing an implant for limiting exogenous subtalar joint motion, comprising:
a handle;
a shaft extending from said handle;
a blade extending from said shaft, said blade including a pin which extends beyond the edge of said blade;
a project extending laterally from said blade, the lateral outermost surface of said projection forming a guiding surface.

14. The osteotome of claim 13, wherein the implant is as defined in one or more of claims 1 to 12.

15. A set comprising the osteotome of claim 13 and an implant, wherein the implant is as defined in one or more of claims 1 to 12.
